(19) Europäisches Patentamt
European Patent Office
Office européen des brevets

(11) EP 4 137 501 A1

(12) EUROPEAN PATENT APPLICATION
published in accordance with Art. 153(4) EPC

(43) Date of publication:
**22.02.2023 Bulletin 2023/08**

(21) Application number: **21789499.7**

(22) Date of filing: **31.03.2021**

(51) International Patent Classification (IPC):
***C07H 21/02*** *(2006.01)* ***C07H 21/04*** *(2006.01)*
***C12N 15/09*** *(2006.01)*

(52) Cooperative Patent Classification (CPC):
**C07H 21/02; C07H 21/04; C12N 15/09;** Y02P 20/55

(86) International application number:
**PCT/JP2021/014017**

(87) International publication number:
**WO 2021/210409 (21.10.2021 Gazette 2021/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.04.2020 JP 2020072234**

(71) Applicant: **SUMITOMO CHEMICAL COMPANY, LIMITED**
**Tokyo 103-6020 (JP)**

(72) Inventor: **MORIYAMA, Yuya**
**Osaka-shi, Osaka 555-0021 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMPOSITION CONTAINING NUCLEIC ACID OLIGOMER

(57) The purpose of the present invention is to provide a stable composition comprising a nucleic acid oligomer having a phosphorothioate bond, and a process for preparing the same composition, and a process for preparing the nucleic acid oligomer efficiently from the composition. The present invention provides a composition comprising a nucleic acid oligomer having a phosphorothioate bond represented by formula (1) (wherein the symbols are defined in the specification), an alkylammonium salt, a water-soluble organic solvent, water, and an additive including at least one compound described in the specification.

EP 4 137 501 A1

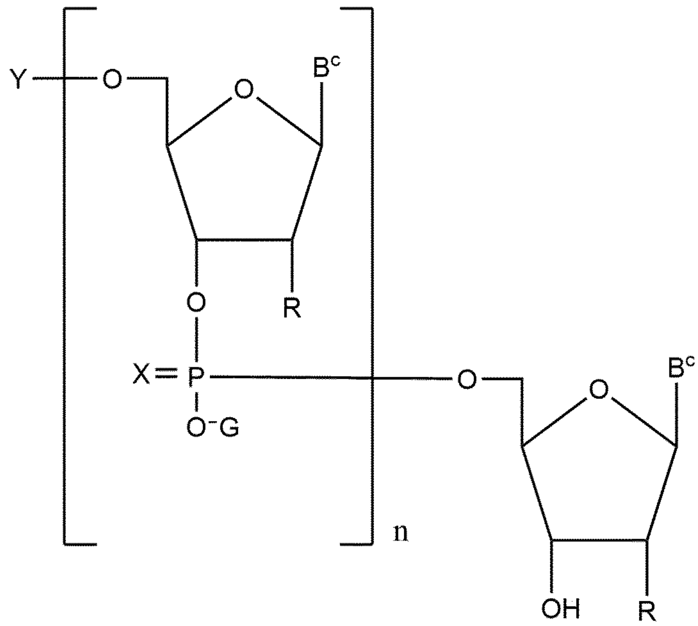

(1)

## Description

### TECHNICAL FIELD

This application claims priority to and the benefit of priority from the Paris Convention based on Japanese Patent Application No. 2020-072234 filed April 14, 2020, the entire contents of which are incorporated herein by reference.
The present invention relates to a composition comprising a nucleic acid oligomer. For more detail, the present invention relates to a composition which comprises a nucleic acid oligomer containing phosphorothioate.
In recent years, there has been growing interest in the application of nucleic acid oligomers to the medical field. For example, an antisense nucleic acid, an aptamer, a ribozyme, and nucleic acids that induces RNA interference (RNAi) such as siRNA are included, which are referred to as a nucleic acid medicine.
It has been publicly known that a nucleic acid oligomer is synthesized by a solid phase synthesis, and a nucleic acid oligomer containing a phosphorothioate bond has also been publicly known as a useful compound which is synthesized by a solid phase method (see patent Literature 1).

### CITATION LIST

### NON-PATENT LITERATURE

Patent Literature 1: WO 2017/068377 A1

### SUMMARY OF THE INVENTION

### (PROBLEMS TO BE SOLVED BY INVENTION)

Nucleic acid oligomers containing a phosphorothioate bond may have any problems in stability during their preparation process. An object of the present invention is to provide a stable composition which comprises a nucleic acid oligomer containing a phosphorothioate bond, a process for preparing the same, and an efficient process for preparing the nucleic acid oligomer from the composition.

### (MEANS TO SOLVE PROBLEMS)

The present inventors have intensively studied to achieve the above object, and as a result, can find out that a crude product of a nucleic acid oligomer containing a phosphorothioate bond produced by the phosphoramidite method of the solid-phase synthesis method is subjected to a reversed-phase chromatography to obtain a nucleic acid oligomer, and the nucleic acid oligomer is mixed with an alkylammonium salt, a water-soluble organic solvent, and water, and certain additives to stabilize the resulting composition. Accordingly, the present invention provides the composition, a process for preparing the same composition, and an efficient process for preparing nucleic acid oligomer from the composition.
The present invention encompasses the following aspects, but are not limited thereto.

[1] A composition comprising a nucleic acid oligomer having a phosphorothioate bond represented by formula (1):

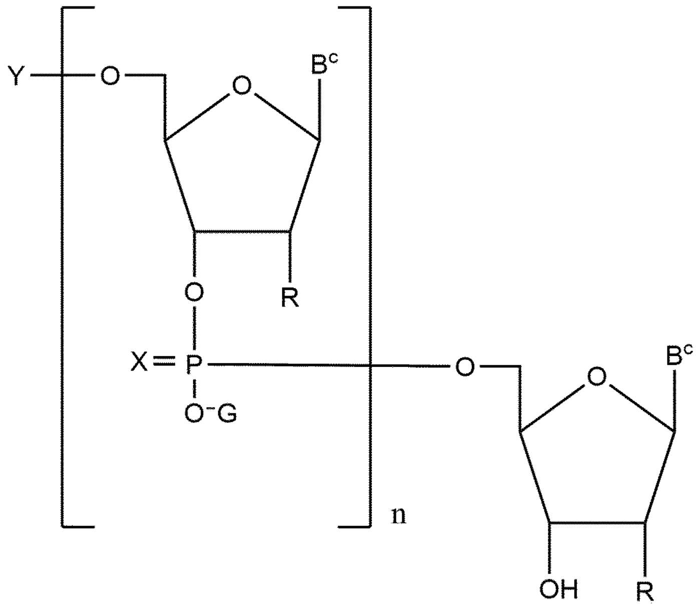

(1)

wherein

$B^c$ is the same or different from each other and each represents independently a nucleic acid base,
R is the same or different from each other and each represents independently a hydrogen atom, a fluorine atom, or OQ group,
Q is the same or different from each other and each represents a hydrogen atom, a methyl group, a 2-methoxyethyl group, a methylene group that is attached to a carbon atom at a 4'position of ribose, an ethylene group that is attached to a carbon atom at a 4'position of ribose, or an ethylidene group that is attached to a carbon atom at a 4'position of ribose,
X is the same or different from each other and each represents an oxygen atom or a sulfur atom,
Y represents a hydrogen atom or a protecting group of hydroxy group,
G represents an ammonium ion, an alkylammonium ion, an alkali metal ion, a hydrogen ion or a hydroxyalkylammonium ion,
n is an integer that satisfies an equation (2):

$$15 \leqq n \qquad (2),$$

an alkylammonium salt, a water-soluble organic solvent, water, and an additive,
the additive is an additive including at least one compound selected from the group consisting of compounds represented by the following formula (3) or (4),
a compound represented by formula (3):

$$R^a(R^c)CH\text{-}L\text{-}CH(R^d)R^b \qquad (3)$$

wherein
L represents -S- or -SS-,
$R^a$ and $R^b$ are the same or different from each other and each represents independently a hydrogen atom, or a C1-6 alkyl group which may be optionally substituted with at least one group selected from a group consisting of the following $Z^1$ and $Z^2$,

$Z^1$: $-CH(NHR^1)COR^2$,

$Z^2$: $-COR^2$

in $Z^1$ and $Z^2$,
$Z^1$ represents a hydrogen atom, a protecting group for amino group, or $C(O)\text{-}R^{11}$ group,

$R^{11}$ represents a C1-6 alkyl group which may be optionally substituted with at least one group selected from a group consisting of amino group and carboxy group, or a phenyl group which may be optionally substituted with at least one group selected from a group consisting of amino group and carboxy group,

$R^2$ represents a C1-6 alkylimino group which may be optionally substituted with carboxy group, wherein the carboxy group may be optionally protected, or -$OR^{20}$ group ($R^{20}$ represents a hydrogen atom or a protecting group for carboxy group),

$R^c$ and $R^d$ are the same or different from each other and each represents independently a hydrogen atom, or a C1-6 alkyl group,

a compound represented by formula (4):

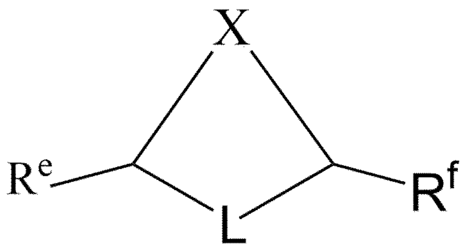

(4)

wherein

L has the same meanings as defined above,

$R^e$ and $R^f$ are the same or different from each other and each represents independently a hydrogen atom, a C1-6 alkoxycarbonyl group, a carboxy group, or a C1-6 alkyl group which may be optionally substituted with C1-6 alkoxycarbonyl group or carboxy group,

X, L and carbon atoms to which they are attached form a 5- membered or 6- membered ring structure, and

X represents any group selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C=N$, $CH_2NH$, $(CH_3)CHNH$, $(CH_3)_2CNH$, (COOH) CHNH, $CH_2OCH_2$, $CH_2NHCH_2$ and $CH_2COCH_2$.

[2] The composition according to [1] wherein

$R^1$ represents a hydrogen atom, a protecting group for amino group, or C(O)-$R^{11}$ group,

$R^{11}$ represents a C1-6 alkyl group that may be optionally substituted with at least one group selected from the group consisting of amino group and carboxy group, and

X represents any groups selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C=N$, $CH_2NH$, $(CH_3)CHNH$, (COOH) CHNH, $CH_2OCH_2$, and $CH_2COCH_2$.

[3] The composition according to [1] or [2] wherein

$R^1$ represents a hydrogen atom, a benzoyl group, a 4-methoxybenzoyl group, a formyl group, an acetyl group, a propionyl group, a butyryl group, an isobutyryl group, a phenylacetyl group, a phenoxyacetyl group, a 4-tert-butyl phenoxyacetyl group, a 4-isopropyl phenoxyacetyl group, a benzyloxy carbonyl group, a 9-fluorenylmethyloxy carbonyl group, or C(O)-$R^{11}$ group,

$R^{11}$ represents a C1-6 alkyl group that may be optionally substituted with at least one group selected from the group consisting of amino group and carboxy group,

$R^2$ represents a C1-6 alkylimino group that may be optionally substituted with carboxy group wherein carboxy group may be protected by methyl group, benzyl group, allyl group or tert-butyl group, or -$OR^{20}$ group ($R^{20}$ represents a hydrogen atom, a methyl group, a benzyl group, an allyl group or a tert-butyl group), and

X represents any groups selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C=N$, $CH_2NH$, (COOH)CHNH, $CH_2OCH_2$, and $CH_2COCH_2$.

[4] The composition according to any one of [1] to [3] wherein

$R^e$ and $R^f$ are the same or different from each other and each independently represents a hydrogen atom, a carboxy group, or a C1-6 alkyl group, and

X represents any groups selected from $CH_2$, $(CH_3)C=N$, $CH_2NH$, $CH_2OCH_2$, and $CH_2COCH_2$.

[5] The composition according to any one of [1] to [4] wherein

$R^1$ represents a hydrogen atom, a benzoyl group, a formyl group, an acetyl group, a benzyloxy carbonyl group and a 9-fluorenylmethyloxy carbonyl group or C(O)-$R^{11}$ group,
$R^{11}$ represents a C1-6 alkyl group that may be optionally substituted with at least one group selected from the group consisting of amino group and carboxy group, and
$R^2$ represents a C1-6 alkylimino group that may be optionally substituted with carboxy group wherein the carboxy group may be optionally protected by methyl group, or -$OR^{20}$ group ($R^{20}$ represents a hydrogen atom or a methyl group) .

[6] The composition according to any one of [1] to [5] wherein

the additives are at least one compound selected from the group consisting of
α-lipoic acid,
methionine,
N-formyl methionine,
N-acetyl methionine,
N-benzoyl methionine,
N-carbobenzoxy methionine,
N-Fmoc-methionine,
methionine methyl ester hydrochloride salt,
dibutyl sulfide,
dihexyl sulfide,
thiazolidine-2-carboxylic acid,
2-isobutyl-4,5-dimethyl-3-thiazoline (isomer mixture),
4-oxothiane,
1,4-thioxane, and
oxidized glutathione.

[7] The composition according to any one of [1] to [6] wherein the additives are at least one compound selected from the group consisting of lipoic acid, oxidized glutathione and methionine.
[8] The compound according to any one of [1] to [7] wherein the alkylammonium salt is at least one of alkylammonium salt selected from the group consisting of monoalkylammonium salts and dialkylammonium salts.
[9] The composition according to any one of [1] to [8] wherein the water-soluble organic solvent is a water-soluble organic solvent selected from the group consisting of alcoholic water-soluble organic solvents and nitrile water-soluble organic solvents.
[10] The composition according to any one of [1] to [9] wherein in the above formula (1), R each independently represents a hydroxy group or a methoxy group.
[11] The composition according to any one of [1] to [9] wherein in the above formula (1), R represents a hydroxy group.
[12] A process for preparing a nucleic acid oligomer which comprises a step of mixing the composition according to any one of [1] to [11] with a C1-C4 organic solvent containing at least one oxygen atom to isolate a precipitated nucleic acid oligomer.
[13] A process for preparing the composition according to any one of [1] to [12] which comprises a step of mixing column eluate containing a nucleic acid oligomer represented by formula (1) wherein the nucleic acid oligomer is obtained by subjecting a crude product of a nucleic acid oligomer of formula (1) that is synthesized by a solid-phase synthesis method to a reversed-phase chromatography, an alkylammonium salt, a water-soluble organic solvent and water,
with an additive.

Effect of Invention

The present invention provides a stable composition comprising a nucleic acid oligomer containing a phosphorothioate bond, and an effective process for preparing the nucleic acid oligomer using the same composition.

Brief Description of Drawings

[Fig. 1]
Figure 1 is a figure showing an example of a synthesis of nucleic acid oligomer by phosphoramidite method.

MODE FOR CARRYING OUT THE INVENTION

It explains a composition comprising a nucleic acid oligomer containing a phosphorothioate bond represented by the formula (1), an alkylammonium salt, a water-soluble organic solvent, water, and additives, wherein the additives represent at least one compound selected from the group consisting of a compound containing a disulfide bond, and a compound containing a sulfide bond.

In the formula (1), a nucleic acid base represented by $B^c$ (hereinafter, referred to as "base") may be a natural or non-natural nucleic acid base. Examples of the non-natural nucleic acid base include modified analogues of the naturally occurring or the non-naturally occurring nucleic acid bases. Typical examples of the nucleic acid base include purine compounds and pyrimidine compounds, and include, for example, nucleic acid bases disclosed in each of U.S. Patent No. 3,687,808; "Concise Encyclopedia Of Polymer Science And Engineering, pp. 858-859, edited by Kroschwitz J.I., John Wiley & Sons, 1990; and Englisch et al., Angewandte Chemie, International Edition, 1991, vol. 30, p. 613.

Specific examples of the nucleic acid base include purine bases such as adenine, isoguanine, xanthine, hypoxanthine and guanine; and pyrimidine bases such as cytosine, uracil and thymine; and the like.

Examples of the nucleic acid base represented by $B^c$ include further include amino derivatives such as 2-aminoadenine, 2-aminopurine, and 2,6-diaminopurine; alkyl derivatives such as 5-methyluracil, 5-methylcytosine, 7-methylguanine, 6-methylpurine, 2-propylpurine; 5-halouracil and 5-halocytosine; 5-propynyluracil and 5-propynylcytosine; 6-azauracil, 6-azacytosine and 6-azathymine; 5-uracil (pseudo uracil), 4-thiouracil, 5-(2-aminopropyl)uracil, and 5-aminoallyluracil; 8-substituted purines, for example, 8-halogenated, aminated, thiolated, thioalkylated or hydroxylated purine, or other 8-substituted purine; 5-trifluoromethylated pyrimidine, or other 5-substituted pyrimidine; 6-azapyrimidine; N-2, N-6 or O-6 substituted purines (including 2-aminopropyladenine); dihydrouracil; 3-deaza-5-azacytosine; 7-deazaadenine; N6-methyladenine, N6,N6-dimethyladenine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 2-thiouracil, 5-methyl-2-thiouracil; 5-methoxycarbonylmethyl-2-thiouracil; 5-methylaminomethyl-2-thiouracil, 3-(3-amino-3-carboxypropyl)uracil; 3-methylcytosine; N4-acetylcytosine; 2-thiocytosine; N6-methyladenine; N6-isopentyladenine; 2-methylthio-N6-isopentenyladenine; N-methyl guanine; O-alkylated bases, or the like.

When R represents OQ group, and Q represents a methylene group that is attached to a carbon atom at 4' position of ribose, an ethylene group that is attached to a carbon atom at 4' position of ribose, or an ethylidene group that is attached to a carbon atom at 4' position of ribose, the structures of the nucleotides are represented by LNA-1, LNA-2, and LNA-3 structures represented by the following formulae (3) .

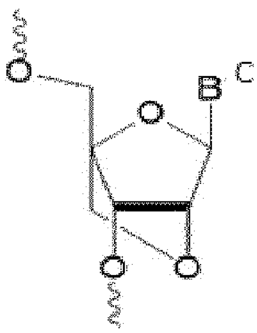
LNA-1

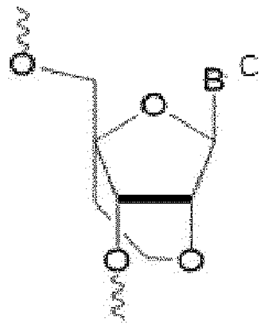
LNA-2

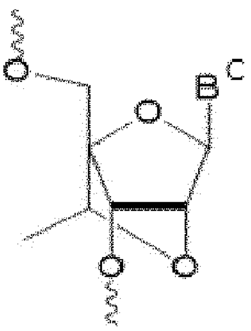
LNA-3

(3)

(wherein $B^c$ represents a nucleic acid base as mentioned above)

The protecting group of hydroxy group represented by Y may be any groups without any particular limitations as long as they may function as a protecting group in an amidite method, and for example, the publicly known protecting group that is used in an amidite compound may be widely used. Examples of the protecting group of hydroxy group represented by Y includes preferably the following groups.

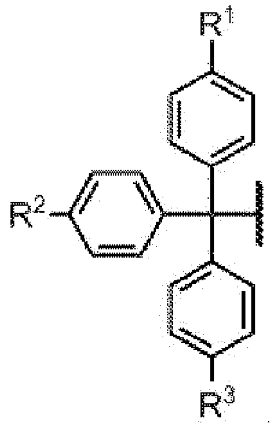

(wherein $R^1$, $R^2$ and $R^3$ are the same or different from each other and each independently represents a hydrogen atom or an alkoxy group).

Examples of the alkoxy group include a methoxy group.

The strand length of the nucleic acid oligomer of formula (1) is ≧ 15. Examples of the upper limit of the strand length include n ≦ 200. In the above mentioned nucleic acid oligomer, among a number of n of X, at least one of X represents a sulfur atom, and all of X may be sulfur atoms. For example, in the case of n = 103, examples of the number of a sulfur atom include 6, 12, or 20.

The nucleic acid oligomer of formula (1) may be, for example, DNA or RNA oligomer, or those containing non-natural nucleic acid base in these oligomers. Typical examples of the nucleic acid oligomer include a single-stranded DNA or RNA oligomer. In the nucleic acid oligomer of the formula (1), preferably the substituent R each independently represents a hydroxy group or a methoxy group. Examples of the nucleic acid oligomer include preferably RNA which is a nucleic acid oligomer of formula (1) wherein the substituent R each independently represents a hydroxy group or a methoxy group. For more detail, examples of the nucleic acid oligomer include preferably the nucleic acid oligomer which comprises both a nucleotide having a hydroxy group as the substituent R and a nucleotide having a methoxy group as the substituent R.

The concentration of the nucleic acid oligomer in the composition is within a range of usually 0.05 mg/mL to 5 mg/mL, preferably 0.05 mg/mL to 1 mg/mL, and more preferably 0.1 mg/mL to 0.5 mg/mL.

Examples of the alkylammonium salt to be used include usually a monoalkylammonium salt, a dialkylammonium salt and a trialkylammonium salt, preferably a monoalkylammonium salt and a dialkylammonium salt, and more preferably a dialkylammonium salt. The number of carbon atoms in the monoalkyl amine that forms the monoalkylammonium salt is preferably 3 to 10, more preferably 4 to 6, and a hexylamine is further preferably included. The number of carbon atoms in the dialkyl amine that forms the dialkylammonium salt is preferably 4 to 10, and more preferably 5 to 9. Preferable examples of dialkylamine include dibutylamine. The examples of trialkylamine that forms the trialkylammonium salt preferably includes those having 6 to 12 carbon atoms, and more preferably those having 6 to 9 carbon atoms, and specific examples of the trialkylamine include triethylamine.

Examples of acid that forms the monoalkylammonium salt, the dialkylammonium salt and the trialkylammonium salt include carbonic acid, acetic acid, formic acid, trifluoroacetic acid, and propionic acid.

The concentration of the ammonium salt is within a range of usually 1 to 200 mM, preferably 5 to 150 mM, and more preferably 20 to 100 mM.

Examples of the water-soluble solvent include alcoholic organic solvents, and nitrile organic solvents. The amount of the alcoholic organic solvent in the composition is within a range of usually 0 to 20 %, preferably 0 to 15 %, and more preferably 0 to 10 %. The eluate fractions obtained by a reversed-phase column chromatography contain usually water, water-soluble solvent (such as alcoholic organic solvent, and nitrile organic solvent), an alkylammonium salt, and a nucleic acid oligomer of formula (1). The amount of the nitrile organic solvent in the eluates is within a range of usually 10 to 70 %, preferably 20 to 60 %, and more preferably 30 to 50 % (herein "%" represents "mass %").

The amount of water may be any amount that balances to satisfy the concentration range of each of the above mentioned ingredients, and is within a range of usually 90 % to 30 %, preferably 80 % to 40 %, and more preferably 70 % to 40 %.

An additive represented by the formula (3) is described below.

In $Z^1$, a protecting group for amino group represented by $R^1$ is not particularly limited unless specified, and may use publicly known protecting groups. Specific examples of the protecting group include a benzoyl group, a 4-methoxybenzoyl group, a formyl group, an acetyl group, a propionyl group, a butyryl group, an isobutyryl group, a phenylacetyl group, a phenoxy acetyl group, a 4-tert-butyl phenoxy acetyl group, a 4-isopropyl phenoxy acetyl group, a

benzyloxy carbonyl group, and a 9-fluorenylmethyloxy carbonyl group (Fmoc group). Preferable protecting group includes a benzoyl group, a formyl group, a benzyloxy carbonyl group, and a 9-fluorenylmethyloxy carbonyl group.

Examples of the C1-6 alkyl group that may be optionally substituted with at least one group selected from the group consisting of amino group and carboxy group which is represented by $R^{11}$ include $(CH_2)_2CH(NH_2)(COOH)$ group, and examples of the phenyl group that may be optionally substituted with at least one group selected from the group consisting of amino group and carboxy group include a phenyl group, an aminophenyl group, and a carboxyphenyl group.

Preferable examples of $R^{11}$ include a C1-6 alkyl group that may be optionally substituted with at least one group selected from the group consisting of amino group and carboxy group (such as $(CH_2)_2CH(NH_2)(COOH)$ group) .

In $Z^1$ and $Z^2$, examples of $COR^2$ group include a COOH group and a $COOR^{20}$ group, and the protecting group of the carboxy group represented by $R^{20}$ is not particularly limited, and publicly known protecting groups may be used, and examples of the protecting groups include a methyl group, a benzyl group, an allyl group, and a tert-butyl group.

Examples of the C1-6 alkylimino group that may be optionally substituted with carboxy group wherein the carboxy group may be optionally protected, which is represented by $R^2$ include a methylimino group, an ethylimino group, a propylimino group, a buthylimino group, a pentylimino group, and a hexylimino group, or those groups in which these groups are substituted with a carboxy group wherein the carboxy group may be optionally protected by methyl group, benzyl group, allyl group or tert-butyl group. The C1-6 alkylimino group that may be optionally substituted with carboxy group wherein the carboxy group may be optionally protected by methyl group is preferably included, and preferable examples of the C1-6 alkylimino group include $NHCH_2CO_2H$ group.

Specific examples of the additives represented by formula (3) include methionine, oxidized glutathione, N-formyl methionine, N-acetyl-DL-methionine, N-benzoyl-DL-methionine, N-carbobenzoxy-DL-methionine, N-Fmoc-L-methionine, L-methionine methyl ester hydrochloride salt, dibutyl sulfide, and dihexyl sulfide.

Next, the definitions of the compound represented by formula (4) is described.

First, in the groups represented by $R^e$ and $R^f$, examples of a C1-6 alkyl group or an alkyl part which constitutes the C1-6 alkoxycarbonyl group and the C1-6 alkyl group include a methyl group, an ethyl group, a propyl group, an isobutyl group, a n-butyl group, a pentyl group and a hexyl group.

Examples of the group represented by $R^e$ and $R^f$ include preferably a hydrogen atom, a carboxy group, and a C1-6 alkyl group.

Specific examples of the group represented by $R^e$ and $R^f$ include a carboxy group, an isobutyl group, $(CH_2)_4CO_2H$, and $CO_2C_2H_5$.

Examples of "X" include the above mentioned groups as described in formula (4), and preferable examples of "X" include $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C=N$, $CH_2NH$, $(CH_3)CHNH$, (COOH)CHNH, $CH_2OCH_2$, and $CH_2COCH_2$, and more preferable examples of "X" include $CH_2$, $(CH_3)C=N$, $CH_2NH$, $CH_2OCH_2$ and $CH_2COCH_2$.

Specific examples of the compound represented by formula (4) include α-lipoic acid, thiazolidine-2-carboxylic acid, 2-isobutyl-4,5-dimethyl-3-thiazoline (isomer mixture).

These additives may be usually used as an aqueous solution or a solution in a water-soluble organic solvent.

The concentration of the additives is within a range of usually 0.1 μM to 100 mM, and preferably 1 mM to 10 mM.

The additives are available by the methods disclosed in JP Patent No. 4,476,386 B1, JP Patent No. 5,317,836 B1, "Fundamentals of modern peptide synthesis", pages 3 to 24, John Howl, edit., Muriel Amblard, Jean-Alain Fehrentz, Jean Martinez, Methods in Molecular Biology (Trademark), book series, volume 298, 2005, in addition to purchasing.

The composition of the present invention may be usually prepared by subjecting the crude product of nucleic acid oligomer of formula (1) that is synthesized by solid phase synthesis to a reversed-phase column chromatography treatment using a mobile phase containing an alkylammonium salt, a water-soluble organic solvent and water to obtain column eluate, followed by adding the above mentioned additives to the column eluate. Alternatively, the composition of the present invention may be prepared by subjecting the crude product of nucleic acid oligomer to a reversed-phase column chromatography treatment using a mobile phase which contains the additives in advance to obtain the desired composition as an eluate fraction of the reversed-phase column chromatography.

The eluate fraction that is obtained by a reversed-phase column chromatography is analyzed on its constitution with UV absorption at wavelength 260 nm under a chromatography condition that is commonly used for separation analysis of nucleic acid, and then is selected and collected. As a purified product, the desired nucleic acid oligomer having prescribed amount of phosphorothioate bond is obtained from the collected fractions. As the analysis method, for example, the method described in non-patent literature (Handbook of Analysis of Oligonucleotides and Related Products, CRC Press) may be used.

Examples of the fillers for the reversed-phase column chromatography include as a silica or a polymer that works as hydrophobic stationary phase a silica or a polymer on which one or more kinds of groups selected from a phenyl group, an alkyl group having 1 to 20 carbon atoms or a cyanopropyl group is fixed. Examples of the silica or the polymer to be used as a filler include those having particle size of 2 um or more, or 5 um or more.

As the mobile phase of reversed-phase column chromatography, for example, it is used a mobile phase containing an aqueous solution of ammonium salt having the above mentioned concentration and pH, and a mobile phase in which the above mentioned water-soluble organic solvent is contained, and a gradient of successively increasing the concentration is applied. The temperature of the reversed-phase column chromatograph is within a range of usually 20 to 100°C, preferably 30 to 80°C, and more preferably 40 to 70°C. The composition of the present invention can be typically obtained as the above mentioned eluate fraction of reversed-phase column chromatography.

The composition of the present invention may be subjected after a storage step to a single step or a plural of steps of work-up steps including a reprecipitation step, a separation step with a separatory funnel step, a ultrafiltration step, a deprotection step, and a lyophilization step, for isolating a nucleic acid oligomer. In the storage step, an atmosphere in a storage container may be replaced with inert gas. Examples of the inert gas include nitrogen gas, argon gas, and helium gas.

In the reprecipitation step, a stabilized solution may be contacted with a poor solvent to precipitate out and isolate a nucleic acid oligomer. If necessary, the liquid parts may be removed from a solid-liquid separation state, and the precipitated nucleic acid oligomer may be collected and isolated by filtration etc. Examples of the poor solvent in the reprecipitation step include a C1-C4 organic solvent containing at least one oxygen atom (such as C1-C4 alcohols, tetrahydrofuran, dioxane). Preferable examples of the solvent include ethanol an isopropanol.

In the separation step with a separatory funnel, a stabilized solution is mixed with at least one kind solvent selected from an aqueous acidic solution such as aqueous acetic acid solution, water or brine, and thereto is added by organic solvent that is immiscible with water, and the resulting mixture is separated with a separatory funnel to an aqueous layer and an organic layer to obtain an aqueous layer containing a desired nucleic acid oligomer.

In the ultrafiltration step, an ultrafiltration membrane is used to separate a nucleic acid oligomer that is existed in the solution after the storage step from lower molecular weight components having the desired molecular weight or less.

In the case where a protecting group is present at the 5'position in the end of the nucleic acid oligomer, in order to deprotect the protecting group, the solution after the storage step is mixed with an aqueous acidic solution such as aqueous acetic acid solution or a solution of acidic substance such as acetic acid that is solubilized in organic solvent to deprotect the protecting group of nucleic acid oligomer.

In the lyophilization step, a frozen aqueous solution of nucleic acid oligomer is set under reduced pressure to sublime water, and separate the nucleic acid oligomer from moisture.

For the synthesis of nucleic acid oligomer by a phosphoramidite method, a nucleic acid elongation reaction can be carried out according to a publicly known method (for example, a method described in the JP patent No. 5,157,168 B1, or JP patent No. 5,554,881 B1). For the preparation of nucleic acid oligomer by a phosphoramidite method, a process for preparing nucleic acid oligomer is described by taking a synthesis of RNA of the scheme indicated in Fig.1 as an example, and referring to the below-mentioned reaction pathway (coupling reaction, oxidation, and deprotection).

In the above mentioned scheme indicating a reaction pathway, $B^a$ represents an optionally-protected nucleic acid base; Tr represents a protecting group; X is as defined above, and SP represents a part other than nucleoside structure of inorganic porous carrier.

A nucleic acid base that is composed of inorganic porous carrier having nucleoside structure (Sp-Nu) and a nucleotide of amidite monomer (Am-1) represents the above mentioned nucleic acid base or an nucleic acid base that is protected by a protecting group.

Preferable examples of the amidite monomer (Am-1) include TBDMS amidite (TBDMS RNA Amidites, product name, ChemGenes Corporation), ACE amidite, TOM amidite, CEE amidite, CEM amidite, TEM amidite (Reviews by Chakhmakhcheva: Protective Groups in the Chemical Synthesis of Oligoribonucleotides, Russian Journal of Bioorganic Chemistry, 2013, Vol. 39, No. 1, pp. 1-21.), and EMM amidite (as described in WO2013/027843 A1), or the like, in which when R represents a protected hydroxy group in the compound represented by the following chemical formula (Am-1'), the specific protecting group is tert-butyldimethylsilyl (TBDMS) group, bis(2-acetoxy)methyl (ACE) group, (triisopropylsilyloxy)methyl (TOM) group, (2-cyanoethoxy)ethyl (CEE) group, (2-cyanoethoxy)methyl (CEM) group, para-tolylsulfonylethoxymethyl (TEM) group, (2-cyanoethoxy)methoxymethyl (EMM) group, or the like.

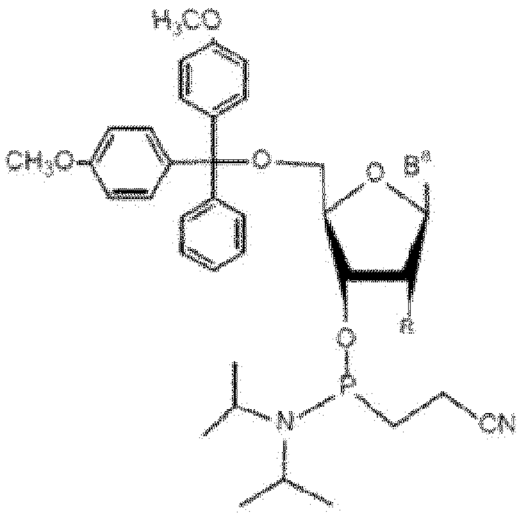

(Am-1')

(wherein R represents the above mentioned group, and $B^a$ represents a nucleic acid base that may be optionally protected.)

[Solid phase synthesis of RNA]

A Tr group of the inorganic porous carrier (Sp-Nu) is deprotected to obtain the solid-phase carrier (Am-2). Then, the amidite monomer (Am-1) and the solid-phase carrier (Am-2) are subjected to a coupling reaction to obtain a reaction product (Am-3). Then, the reaction product (Am-3) is oxidized to obtain the product (Am-4). Then, the product (Am-4) is deprotected (-Tr) to obtain the product (Am-5). Then, the amidite monomer (Am-1) and the product (Am-5) are further subjected to a coupling reaction to elongate the phosphodiester bond. As described above, the hydroxy group of the S'position at the end of the elongated oligonucleotide strand is repeatedly subjected to a series of cycle including deprotection, coupling reaction and oxidation as many times as necessary so as to provide a desired sequence, and then the resulting product can be cleaved from the solid-phase carrier to produce a nucleic acid molecule having a desired sequence. The synthesis may be carried out by means of an automatic nucleic acid synthesizer or the like that employs the phosphoramidite method. Here RNA is explained by taking it as an example, and the explanations can be applied to nucleic acid compounds including nucleotide other than ribonucleotide.

In the step of deprotecting Tr group, a protecting group of hydroxy group of the S'position at the end of the RNA that is supported on the solid carrier is deprotected. Examples of the protecting group to be used include trityl -based protecting group (typically DMTr group). The deprotection can be carried out with an acid. Examples of the acid for deprotection include trifluoroacetic acid, trichloroacetic acid, dichloroacetic acid, trifluoromethanesulfonic acid, methanesulfonic acid, hydrochloric acid, acetic acid, and p-toluenesulfonic acid.

In the coupling step, the nucleoside phosphoramidite is bound to the hydroxy group at the S'position in the end of the RNA strand which is deprotected in the deprotection step so as to produce the phosphite. As the nucleoside phosphoramidite, a nucleoside phosphoramidite in which the hydroxy group at the S'position is protected by a protecting group (for example, DMTr group) is used.

Also, the coupling step can be carried out with an activator which activates the nucleoside phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methylbenzimidazolium triflate (N-MeBIT), benzimidazolium triflate (BIT), N-phenylimidazolium triflate (N-PhIMT), imidazolium triflate (IMT), 5-nitrobenzimidazolium triflate (NBT), 1-hydroxybenzotriazole (HOBT), 5-(bis-3,5-trifluoromethylphenyl)-1H-tetrazole (Activator-42), and the like.

After the coupling step, an unreacted hydroxy group at the S'position may be capped as needed. The capping can be carried out with a publicly known capping solution such as acetic anhydride-tetrahydrofuran solution, phenoxyacetic acid anhydride / N-methyl imidazole solution, and the like.

The oxidation step refers to a step of oxidizing the phosphite formed in the coupling step. The oxidation step can be carried out with an oxidizing agent. Examples of the oxidizing agent include iodine, m-chloroperbenzoic acid, tert-butylhydroperoxide, 2-butanoneperoxide, bis(trimethylsilyl)peroxide, 1,1-dihydroperoxycyclododecane, hydrogen peroxide, and the like.

When a phosphite triester group is converted into a thiophosphate triester group, as "oxidation agent", for example, a sulfur, 3H-1,2-benzodithiol-3-one-1,1-dioxide (Beaucage reagent), 3-amino-1,2,4-dithiazole-5-thione

(ADTT), 5-phenyl-3H-1,2,4-dithiazole-3-one (POS), [(N,N-dimethylaminomethylidene)amino]-3H-1,2,4-dithiazoline-3-thione (DDTT), and phenylacetyldisulfide (PADS) can be used. The oxidation agent can be used by diluting it with an appropriate solvent so as to adjust to 0.001 to 2 M of the concentration of the agent. The solvents to be used are not particularly limited as long as they do not involve the reaction, and include, for example, dichloromethane, acetonitrile, pyridine, or any mixed solvents of these solvents at arbitrary ratio thereof.

The oxidation step may be carried out after the above mentioned capping procedure, or vice versa, the capping procedure may be carried after the oxidation step, and the order of the procedures is not limited.

After the oxidation step, the procedure is returned to a deprotection step, and the above mentioned series of cycle steps including coupling reaction, oxidation, and deprotection is repeatedly carried out depending on the nucleotide sequence of the nucleic acid oligomer to be synthesized to synthesize RNA having a desired sequence.

After the completion of the synthesis of the nucleic acid oligomer having a desired sequence, the RNA strand is cleaved and recovered from the solid carrier using ammonia or amine compound.

Examples of the amine compound include methylamine, ethylamine, isopropylamine, ethylenediamine, diethylamine, and triethylamine.

The strand length of the nucleic acid oligomer that is obtained by the above process is, for example, within a range of $n \geqq 60$, $n \geqq 80$, or $n \geqq 100$, and also $n \leqq 200$. The strand length of the nucleic acid oligomer include preferably $n \geqq 60$. Specific examples of the strand length of the nucleic acid oligomer include n = 67, 100 or 120.

In the step of deprotecting phosphate protecting group, after the completion of the synthesis of nucleic acid having the desired sequence, an amine compound is acted so as to deprotect the protecting group of phosphate part. Examples of the amine compound include diethylamine as described herein.

In the case where a protecting group for hydroxy group at 2'position or 3'position of ribose is present, the protecting group can be removed according to a method described in WO 2006/022323 A1, WO 2013/027843 A1, or WO 2019/208571 A1.

EXAMPLES

Hereinafter, the present invention is explained in more detail by working examples, and the present invention is not limited to these examples.

Measurement method

Each measurement method used in the following tests is shown below.

(Measurement method 1: measurement method for purity of RNA)

A purity of RNA in the solution after fractionation was measured by HPLC.

The fractionated RNA was separated to each component by HPLC (wavelength 260 nm, column DNAPac TM PA200, 4.0 mm × 250 mm, 8.0 μm), and the purity of RNA was calculated from an area value of peak of major products in the total area value of peaks of the obtained chromatogram.

A HPLC measurement condition is shown in the below Table 1.

[Table 1]

| | |
|---|---|
| Column | DNAPac™ PA200, 4.0 mm × 250 mm, 8.0 um |
| Flow rate | 1.0 mL/min |
| Detection wavelength | 260 nm |
| Mobile phase A | 25mM Tris-HCl buffer (pH 8.0), (containing 10 % acetonitrile, 6M urea) |
| Mobile phase B | 25mM Tris-HCl buffer (pH 8.0), (containing 500mM sodium perchlorate, 10 % acetonitrile, 6M urea) |
| Gradient condition | Bconc. 20%(0 min)-60%(60 min)-90%(60.01 min)-90%(65 min)-20%(65.01min)-20%(80 min) |
| Column temperature | 80°C |

[Reference Example 1]

Solid phase synthesis of RNA by Amidite method

RNA having nucleic acid sequence I represented below was synthesized. The strand length of the RNA consists of 103 base lengths.

Strand I:

A*U*A*ACUCAAUUUGUAAAAAAGUUUUAGAGCUAGAAAUAGCAAGUUAAAAUAAGGCU

AGUCCGU UAU CAACUUGAAAAAGUGGCACCGAGUCGGUGCUUUU*U*U*U(5'-3')

(Sequence No. 1)

In the indication of the above sequence, the symbol of * between the nucleotides represents that the phosphate bond which links nucleotides is a phosphorothioate.

The RNA was synthesized from the 3' end towarding to 5' end with a nucleic acid synthesizer (AKTA oligopilot plus100, manufactured by GE Healthcare) according to a phosphoramidite method. The synthesis was carried out in 63 μmol scale. Also for the synthesis, a uridine EMM amidite (described in Example 2 of WO 2013/027843 A1), a cytidine EMM amidite (described in Example 3 of the same), an adenosine EMM amidite (described in Examples 4 of the same), and a guanosine EMM amidite (described in Example 5 of the same), each of which is represented by the following formula respectively were used as RNA amidites, and a porous glass was used as a solid carrier, and dichloroacetic acid in toluene solution was used as a deblocking solution, and 5-benzyltho-1H-tetrazole was used as a condensing agent, an iodine solution was used as an oxidizing agent, and 3-amino-1,2,4-dithiazole-5-thione was used as a sulfurizing agent, and a phenoxyacetic anhydride solution and N-methyl imidazole solution were used as a capping solution. After a completion of a nucleic acid elongation, diethylamine solution was acted on the nucleic acid on the carrier to deprotect selectively a cyanoethyl protecting group of a phosphate part. As herein, "EMM" represents an abbreviation of (2-cyanoethoxy)methoxymethyl group.

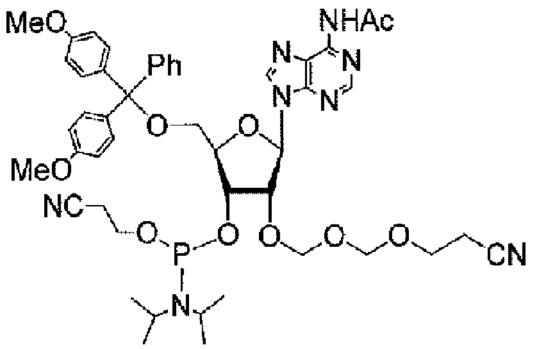

adenosine EMM amidite

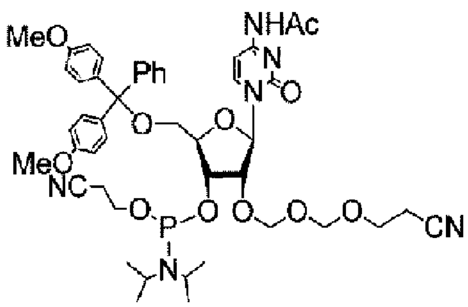

cytidine EMM amidite

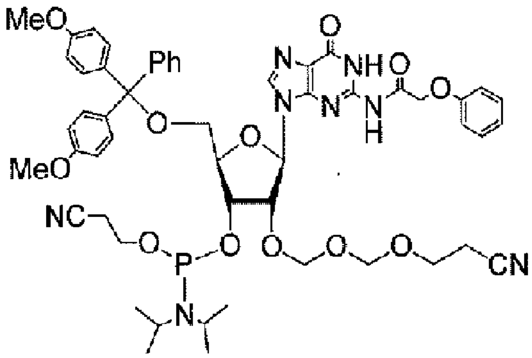

guanosine EMM amidite

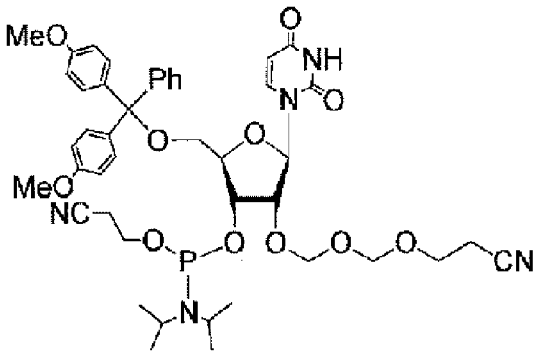

uridine EMM amidite

The cleavage from the solid carrier and the deprotection after a solid synthesis were carried out according to the method described in WO 2013/027843 A1. Specifically, aqueous ammonia solution and ethanol were added, and the mixture was left to stand for a while, and the solid carrier was then filtered, and the solvent was evaporated off. After that, the deprotection of a hydroxy group was carried out with tetrabutylammonium fluoride. The obtained RNA was dissolved with a distilled water for injection to adjust the desired concentration thereof.

Preparative purification of RNA

A column chromatography purification was carried out under the condition of Table 2 below, provided that before the purification, a mobile phase A was passed through the column at a flow rate of 4.7 mL/min for 12.5 min, and the sample was then added. The fractions at retention time of 94.2 min. - 95.8 min. were collected, and the obtained solutions were analyzed by HPLC. Here the purity thereof was calculated according to the method described in the above-mentioned measurement method 1. As a result, the purity was 94.2 %. Using the RNA solution obtained by the preparative purification, the following experiments of the Examples and Comparative Examples were carried out.

[Table 2]

| | |
|---|---|
| Device | AKTA purifier 100 (manufactured by GE Healthcare) |
| Detector | UV 260 nm |
| Column | Triart C18 10mm × 250mm, 10pm (manufactured by YMC) |
| Separation Mode | Reversed-phase chromatography |
| Column temperatur e | 60°C |
| Flow rate | 4.7 mL/min |
| Mobile phase A | 100 mM Aqueous dibutylammonium acetate solution (pH 7.0) |
| Mobile phase B | Acetonitrile |
| Fraction width | 3.9 mL |
| Gradient condition | B conc. 0%(0min)-30%(12.5min)-40%(95.8min)-100%(95.9min)-100%(104min) |

[Example 1]

Ninety nine (99) μL of the RNA solution that was obtained by a preparative purification by a reversed-phase column chromatography in the Reference Example 1 was placed in a volume 300 mL of polypropylene vial (manufactured by Thermo Fisher Scientific), and the solution was mixed with 1 μL of a solution of lipoic acid in acetonitrile as an additive solution to prepare the prescribed concentration of the sample. The vial containing the mixture solution was placed in an incubator (manufactured by Kenis Limited) in which the temperature was adjusted to 60°C, and the vial was left to stand for 8 hours. After left to stand, the polypropylene vial taken out from the incubator was cooled to room temperature, and the purity was calculated by the method described in the above-mentioned measurement method 1. The results are shown in Table 3.

The composition wherein the concentration of the lipoic acid was adjusted to 3 mM (0.07 %) has a constitution composition on the basis of the calculation. Water: 63.79 %, acetonitrile: 34.89 %, dibutylamine 0.84 %, acetic acid: 0.39 % (1.23 % as dibutylammonium acetate); and nucleic acid concentration: 0.21 mg/mL (0.02 %).

[Example 2]

The experiment was carried out under a similar condition to those of the Example 1 wherein an aqueous solution of methionine was used as an additive solution instead of the solution of lipoic acid in acetonitrile to prepare the solution of methionine with a concentration of 3 mM (0.05 %), and the RNA purity after the experiment was measured. The results are shown in Table 3.

[Example 3]

The experiment was carried out under a similar condition to those of the Exmaple 1 wherein an aqueous oxidized glutathione solution was added as an additive solution instead of the solution of lipoic acid in acetonitrile to prepare the solution of oxidized glutathione with a concentration of 0.15 mM (0.01 %), and the RNA purity after the experiment was

measured. The results are shown in Table 3.

[Comparison Example 1]

One hundred (100) μL of the RNA solution that was obtained by a preparative purification by a reversed-phase column chromatography in the Reference Example 1 was placed in a volume 300 mL of polypropylene vial (manufactured by Thermo Fisher Scientific), and the vial containing the solution was placed in an incubator (manufactured by Kenis Limited) in which the temperature was adjusted to 60°C, and the vial was left to stand for 8 hours. After left to stand, the polypropylene vial taken out from the incubator was cooled to room temperature, and the purity was calculated by the method described in the above-mentioned measurement method 1. The results are shown in Table 3.

[Comparison Example 2]

The experiment was carried out under a similar condition to those of the Exmaple 1 wherein each of an aqueous cysteine solution, a solution of N-acetylcysteine in acetonitrile, an aqueous solution of reduced glutathione, an aqueous solution of dithiothreitol, an aqueous solution of L-ascorbic acid, or an aqueous solution of sodium hydrosulfite was used as an additive solution in a prescribed concentration respectively instead of the solution of lipoic acid in acetonitrile. The results are shown in Table 3.

[Table 3]

| | Additives | Concentration of Additives (mM) | Purity by HPLC measurement 1) | Retention ratio of Nucleic acid 2) |
|---|---|---|---|---|
| Ex. 1 | α-lipoic acid | 3.0 | 91.3 % | 97 % |
| | α-lipoic acid | 0.3 | 84.1 % | 89 % |
| | α-lipoic acid | 0.03 | 75.0 % | 80 % |
| Ex. 2 | L-methionine | 3.0 | 93.2 % | 99 % |
| Ex. 3 | Oxidized glutathione | 0.15 | 74.3 % | 79 % |
| Comparative Ex.1 | no additive | - | 66.2 % | 70 % |
| Comparative Ex.2 | L-cysteine | 3.0 | 58.5 % | 62 % |
| | N-acetylcysteine | 3.0 | 16.5 % | 18 % |
| | Reduced glutathione | 0.15 | 38.7 % | 41 % |
| | Dithiothreitol | 3.0 | 6.4 % | 7 % |
| | L-ascorbic acid | 3.0 | 10.8 % | 11 % |
| | Sodium hydrosulfite | 3.0 | 25.2 % | 27 % |
| 1) which represents a purity of nucleic acid that was obtained by leaving the nucleic acid (Purity 94.2 %) prepared in the Reference Exmaple 1 to stand at 60°C for 8 hours. | | | | |

2) Retention ratio of nucleic acid (%) = Nucleic acid purity after left to stand / Nucleic acid purity before left to stand

[Example 4]

(Recovery of RNA from a RNA solution obtained by preparative purification)

In the Example 1, the following treatments were carried out by using the solution in which the lipoic acid was mixed to adjust the concentration thereof to 3 mM and the resulting mixture was left to stand at 60°C for 8 hours. A 80 μL part of the solution was placed in a volume 15 mL of polypropylene conical tube (manufactured by Corning Inc.), and 40 μL of an aqueous solution of sodium acetate (3M, pH = 5.2) and 240 μL of ethanol were added thereto. The resulting slurry solution was centrifuged with 3000 g at 25°C for 10 min., and the supernatant were removed. Successively, 200 μL of 70 % aqueous ethanol solution was placed in the tube, the mixture was centrifuged with 3000 g at 25°C for 10 min., the supernatant was removed, and these steps were repeatedly carried out twice to obtain RNA. The obtained RNA was solubilized in 80 μL water, and the RNA purity was calculated by the method described in the above-mentioned measurement 1 to indicate a purity of 85.8 %.

[Comparison Example 3]

The following treatments were carried out by using the solution in which the resulting mixture was left to stand at 60°C for 8 hours in the comparison Example 1. A 80 μL part of the solution was placed in a volume 15 mL of polypropylene conical tube (manufactured by Corning Inc.), and 40 μL of an aqueous solution of sodium acetate (3M, pH = 5.2) and 240 μL of ethanol were added thereto. The resulting slurry solution was centrifuged with 3000 g at 25°C for 10 min., and the supernatant were removed. Successively, 200 μL of 70 % aqueous ethanol solution was placed in the tube, the mixture was centrifuged with 3000 g at 25°C for 10 min., the supernatant was removed, and these steps were repeatedly carried out twice to obtain RNA. The obtained RNA was solubilized in 80 μL water, and the RNA purity in the fraction was calculated by the method described in the above-mentioned measurement 1 to indicate a purity of 70.9 %.

[Reference Example 2]

Solid phase synthesis of RNA by Amidite method

The RNA having the nucleic acid sequence II represented below was synthesized. The strand length consists of 67 base lengths.

Strand II:

Am*Gm*Cm*AmUmAmGmCAAGUUAmAAAUAAGGmC*U*AmG*U*C*CmGUUAUCAAmCm

UmUmGmAmAmAmAmAmGmUmGGCACmCmGmAGUCGGmUmGmCm*Um*Um*U (5'-3')

(Sequence No. 2)

In the indication of the above sequence, the symbol of * between the nucleotides represents that the phosphate bond which links nucleotides is a phosphorothioate. The alphabets Am, Um, Cm, and Gm each represents a nucleotide wherein the 2'hydroxy group is substituted with a methoxy group. The RNA was synthesized from the 3' end towarding to 5' end with a nucleic acid synthesizer (AKTA oligopilot plus100, manufactured by GE Healthcare) according to a phosphoramidite method. The synthesis was carried out in 53 μmol scale. Also for the synthesis, the uridine EMM amidite (described in Example 2 of WO 2013/027843 A1), the cytidine EMM amidite (described in Example 3 of the same), the adenosine EMM amidite (described in Examples 4 of the same), and the guanosine EMM amidite (described in Example 5 of the same), and an uridine 2'OMe amidite, a cytidine 2'OMe amidite, an adenosine 2'OMe amidite, and a guanosine 2'OMe amidite each of which is represented by the following formula respectively were used as RNA amidites, and a porous glass was used as a solid carrier, and dichloroacetic acid in toluene solution was used as a deblocking solution, and 5-benzylthio-1H-tetrazole was used as a condensing agent, an iodine solution was used as an oxidizing agent, and 3-amino-1,2,4-dithiazole-5-thione was used as a sulfurizing agent, and a phenoxyacetic anhydride solution and N-methyl imidazole solution were used as a capping solution. After a completion of a nucleic acid elongation, diethylamine solution was acted on the nucleic acid on the carrier to deprotect selectively a cyanoethyl protecting group of a phosphate part. As herein, "EMM" represents an abbreviation of (2-cyanoethoxy)methoxymethyl group.

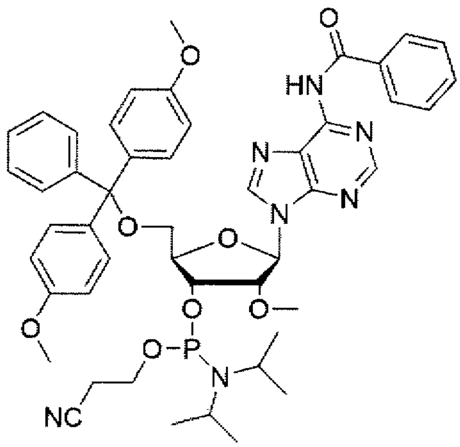

adenosine 2'OMe amidite

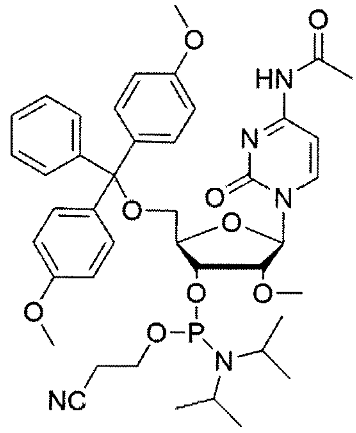

cytidine 2'OMe amidite

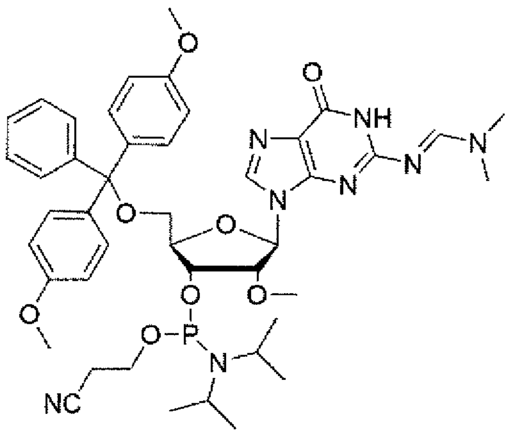

guanosine 2'OMe amidite

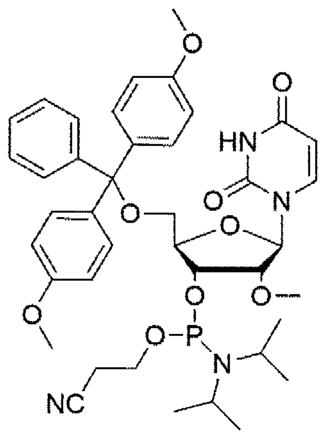

uridine 2'OMe amidite

The cleavage from the solid carrier and the deprotection after a solid synthesis were carried out according to the method described in WO 2013/027843 A1. Specifically, aqueous ammonia solution and ethanol were added, and the mixture was left to stand for a while, and the solid carrier was then filtered, and the solvent was evaporated off. After that, a deprotection of a hydroxy group was carried out with tetrabutylammonium fluoride. The obtained RNA was dissolved with a distilled water for injection to adjust the desired concentration thereof.

Preparative purification of RNA

A column chromatography purification was carried out under the condition of Table 4 below, provided that before the purification, a mobile phase A was passed through the column at a flow rate of 4.7 mL/min for 12.5 min, and the sample was then added. The fractions at retention time of 66.7 min. - 70.9 min. were collected, and the obtained solutions were analyzed by HPLC. Here the purity thereof was calculated according to the method described in the above-mentioned measurement method 1. As a result, the purity was 94.2 %. Using the RNA solution obtained by the preparative purification, the following experiments of the Examples and Comparative Examples were carried out.

[Table 4]

| | |
|---|---|
| Device | AKTA purifier 100 (manufactured by GE Healthcare) |
| Detector | UV 260 nm |
| Column | Triart C18 10mm × 250mm, 10pm (manufactured by YMC) |
| Separation Mode | Reversed-phase chromatography |

(continued)

| | |
|---|---|
| Device | AKTA purifier 100 (manufactured by GE Healthcare) |
| Column temperature | 60°C |
| Flow rate | 4.7 mL/min |
| Mobile phase A | 100 mM Aqueous dibutylammonium acetate solution (pH 7.0) |
| Mobile phase B | Acetonitrile (containing 10% methanol (v/v)) |
| Fraction width | 3.9 mL |
| Gradient condition | B conc. 0%(min)-35%(12.5min)-50%(137.6min)-100%(137.7min)-100%(145.9min) |

[Example 5]

Ninety nine (99) μL of the RNA solution that was obtained by a preparative purification by a reversed-phase column chromatography in the Reference Example 2 was placed in a volume 300 mL of polypropylene vial (manufactured by Thermo Fisher Scientific), and the solution was mixed with 1 μL of an aqueous solution of L-methionine as an additive solution to prepare the 1.5 mM concentration of L-methionine sample. The vial containing the mixture solution was placed in an incubator (manufactured by Kenis Limited) in which the temperature was adjusted to 60°C, and the vial was left to stand for 8 hours. After left to stand, the polypropylene vial taken out from the incubator was cooled to room temperature, and the purity was calculated by the method described in the above-mentioned measurement method 1. The results are shown in Table 5.

The composition wherein the concentration of the L-methionine was adjusted to 1.5 mM (0.02 %) has a constitution composition on the basis of the calculation. Water: 62.95 %, acetonitrile: 32.21 %, methanol: 3.59 %, dibutylamine 0.82 %, acetic acid: 0.38 % (1.20 % as dibutylammonium acetate); and nucleic acid concentration: 0.31 mg/mL (0.03 %).

[Example 6]

The experiment was carried out under a similar condition to those of the Example 5 wherein a solution of N-formyl L-methionine in methanol was used as an additive solution instead of the aqueous solution of L-methionine to prepare the solution of N-formyl N-methionine with a concentration of 3 mM (0.06 %), and the RNA purity after the experiment was measured. The results are shown in Table 5.

[Example 7]

The experiment was carried out under a similar condition to those of the Example 5 wherein a solution of N-acetyl DL-methionine in methanol was used as an additive solution instead of the aqueous solution of L-methionine to prepare the solution of N-acetyl DL-methionine with a concentration of 3 mM (0.06 %), and the RNA purity after the experiment was measured. The results are shown in Table 5.

[Example 8]

The experiment was carried out under a similar condition to those of the Example 5 wherein a solution of N-benzoyl DL-methionine in methanol was used as an additive solution instead of the aqueous solution of L-methionine to prepare the solution of N-benzoyl DL-methionine with a concentration of 3 mM (0.08 %), and the RNA purity after the experiment was measured. The results are shown in Table 5.

[Example 9]

The experiment was carried out under a similar condition to those of the Example 5 wherein a solution of N-carbobenzoxy DL-methionine in methanol was used as an additive solution instead of the aqueous solution of L-methionine to prepare the solution of N-carbobenzoxy DL-methionine with a concentration of 3 mM (0.12 %), and the RNA purity after the experiment was measured. The results are shown in Table 5.

[Example 10]

The experiment was carried out under a similar condition to those of the Example 5 wherein a solution of N-Fmoc-L-methionine in a mixed solvent of methanol and acetonitrile (mixed ratio 50:50 (v/v)) was used as an additive solution instead of the aqueous solution of L-methionine to prepare the solution of N-Fmoc-L-methionine with a concentration of 3 mM (0.10 %), and the RNA purity after the experiment was measured. The results are shown in Table 5.

[Example 11]

The experiment was carried out under a similar condition to those of the Example 5 wherein an aqueous solution of L-methionine methyl ester hydrochloride salt was used as an additive solution instead of the aqueous solution of L-methionine to prepare the solution of L-methionine methyl ester hydrochloride salt with a concentration of 3 mM (0.07 %), and the RNA purity after the experiment was measured. The results are shown in Table 5.

[Example 12]

The experiment was carried out under a similar condition to those of the Example 5 wherein a solution of dibutyl sulfide in acetonitrile was used as an additive solution instead of the aqueous solution of L-methionine to prepare the solution of dibutyl sulfide with a concentration of 3 mM (0.05 %), and the RNA purity after the experiment was measured. The results are shown in Table 5.

[Example 13]

The experiment was carried out under a similar condition to those of the Example 5 wherein a solution of dihexyl sulfide in acetonitrile was used as an additive solution instead of the aqueous solution of L-methionine to prepare the solution of dihexyl sulfide with a concentration of 3 mM (0.07 %), and the RNA purity after the experiment was measured. The results are shown in Table 5.

[Example 14]

The experiment was carried out under a similar condition to those of the Example 5 wherein an aqueous solution of thiazolidine-2-carboxylic acid was used as an additive solution instead of the aqueous solution of L-methionine to prepare the solution of thiazolidine-2-carboxylic acid with a concentration of 3 mM (0.04 %), and the RNA purity after the experiment was measured. The results are shown in Table 5.

[Example 15]

The experiment was carried out under a similar condition to those of the Example 5 wherein a solution of 2-isobutyl-4,5-dimethyl-3-thiazoline (isomer mixture) in acetonitrile was used as an additive solution instead of the aqueous solution of L-methionine to prepare the solution of 2-isobutyl-4,5-dimethyl-3-thiazoline (isomer mixture) with a concentration of 3 mM (0.06 %), and the RNA purity after the experiment was measured. The results are shown in Table 5.

[Example 16]

The experiment was carried out under a similar condition to those of the Example 5 wherein a solution of 4-oxothiane in acetonitrile was used as an additive solution instead of the aqueous solution of L-methionine to prepare the solution of 4-oxothiane with a concentration of 3 mM (0.04 %), and the RNA purity after the experiment was measured. The results are shown in Table 5.

[Example 17]

The experiment was carried out under a similar condition to those of the Example 5 wherein a solution of 1,4-thioxane in acetonitrile was used as an additive solution instead of the aqueous solution of L-methionine to prepare the solution of 1,4-thioxane with a concentration of 3 mM (0.03 %), and the RNA purity after the experiment was measured. The results are shown in Table 5.

[Comparison Example 4]

One hundred (100) μL of the RNA solution that was obtained by a preparative purification by a reversed-phase column chromatography in the Reference Example 2 was placed in a volume 300 mL of polypropylene vial (manufactured by Thermo Fisher Scientific), and the vial containing the solution was placed in an incubator (manufactured by Kenis

Limited) in which the temperature was adjusted to 60°C, and the vial was left to stand for 8 hours. After left to stand, the polypropylene vial taken out from the incubator was cooled to room temperature, and the purity was calculated by the method described in the above-mentioned measurement method 1. The results are shown in Table 5.

[Table 5]

| | Additives | Concentration of Additives (mM) | Purity by HPLC measurement 1) | Retention ratio of Nucleic acid 2) |
|---|---|---|---|---|
| Ex. 5 | L-methionine | 1.5 | 91.5 % | 97 % |
| Ex. 6 | N-formyl L-methionine | 3.0 | 92.0 % | 98 % |
| Ex. 7 | N-acetyl DL-methionine | 3.0 | 92.6 % | 98 % |
| Ex. 8 | N-benzoyl DL-methionine | 3.0 | 92.5 % | 98 % |
| Ex. 9 | N-carbobenzoxy DL-methionine | 3.0 | 92.6 % | 98 % |
| Ex. 10 | N-Fmoc-L-methionine | 3.0 | 92.1 % | 98 % |
| Ex. 11 | L-methionine methyl ester hydrochloride salt | 3.0 | 91.5 % | 97 % |
| Ex. 12 | Dibutyl sulfide | 3.0 | 88.9 % | 94 % |
| Ex. 13 | Dihexyl sulfide | 3.0 | 87.0 % | 92 % |
| Ex. 14 | Thiazolidine-2-carboxylic acid | 3.0 | 92.3 % | 98 % |
| Ex. 15 | 2-isobutyl-4,5-dimethyl-3-thiazoline (isomer mixture) | 3.0 | 90.7 % | 96 % |
| Ex. 16 | 4-Oxothiane | 3.0 | 88.8 % | 94 % |
| Ex. 17 | 1,4-Thioxane | 3.0 | 89.8 % | 95 % |
| Comparative Ex.4 | no additive | - | 83.4 % | 89 % |
| 1) which represents a purity of nucleic acid that was obtained by leaving the nucleic acid (Purity 94.2 %) prepared in the Reference Exmaple 2 to stand at 60°C for 8 hours. | | | | |

2) Retention ratio of nucleic acid (%) = Nucleic acid purity after left to stand / Nucleic acid purity before left to stand.

[Reference Example 3]

Preparative purification of RNA

A column chromatography purification was carried out on the RNA with a hydroxy group being deprotected using tetrabutylammonium fluoride obtained in the Reference Example 2 under the condition of Table 6 below, provided that before the purification, a mobile phase A was passed through the column at a flow rate of 4.7 mL/min for 12.5 min, and the sample was then added. The fractions at retention time of 91.7 min. - 94.2 min. were collected, and the obtained solutions were analyzed by HPLC. Here the purity thereof was calculated according to the method described in the above-mentioned measurement method 1. As a result, the purity was 95.1 %. Using the RNA solution obtained by the preparative purification, the following experiments of the Examples and Comparative Examples were carried out.

[Table 6]

| | |
|---|---|
| Device | AKTA purifier 100 (manufactured by GE Healthcare) |
| Detector | UV 260 nm |

(continued)

| | |
|---|---|
| Device | AKTA purifier 100 (manufactured by GE Healthcare) |
| Column | Triart C18 10mm × 250mm, 10pm (manufactured by YMC) |
| Separation Mode | Reversed-phase chromatography |
| Column temperatur e | 60°C |
| Flow rate | 4.7 mL/min |
| Mobile phase A | 100 mM Aqueous hexylammonium acetate solution (pH 7.0) |
| Mobile phase B | Acetonitrile (containing 10% methanol (v/v)) |
| Fraction width | 3.9 mL |
| Gradient condition | B conc. 0%(0min)-30%(12.5min)-50%(137.6min)-100%(137.7min)-100%(145.9min) |

[Example 18]

Ninety nine (99) μL of the RNA solution that was obtained by a preparative purification by a reversed-phase column chromatography in the Reference Example 3 was placed in a volume 300 mL of polypropylene vial (manufactured by Thermo Fisher Scientific), and the solution was mixed with 1 μL of a solution of α-lipoic acid in acetonitrile as an additive solution to prepare the 3 mM concentration of α-lipoic acid sample. The vial containing the mixture solution was placed in an incubator (manufactured by Kenis Limited) in which the temperature was adjusted to 60°C, and the vial was left to stand for 14 hours. After left to stand, the polypropylene vial taken out from the incubator was cooled to room temperature, and the purity was calculated by the method described in the above-mentioned measurement method 1. The results are shown in Table 7.

The composition wherein the concentration of the α-lipoic acid was adjusted to 3 mM (0.07 %) has a constitution composition on the basis of the calculation. Water: 59.69 %, acetonitrile: 35.38 %, methanol: 3.84 %, hexylamine 0.61 %, acetic acid: 0.36 % (0.97 % as hexylammonium acetate); and nucleic acid concentration: 0.35 mg/mL (0.04 %).

[Example 19]

The experiment was carried out under a similar condition to those of the Example 18 wherein an aqueous solution of L-methionine was used as an additive solution instead of the solution of α-lipoic acid in acetonitrile to prepare the solution of L-methionine with a concentration of 3 mM (0.05 %), and the RNA purity after the experiment was measured. The results are shown in Table 7.

[Example 20]

The experiment was carried out under a similar condition to those of the Example 18 wherein an aqueous solution of DL-methionine was used as an additive solution instead of the solution of α-lipoic acid in acetonitrile to prepare the solution of DL-methionine with a concentration of 3 mM (0.05 %), and the RNA purity after the experiment was measured. The results are shown in Table 7.

[Example 21]

The experiment was carried out under a similar condition to those of the Example 18 wherein an aqueous solution of oxidized glutathione was used as an additive solution instead of the solution of α-lipoic acid in acetonitrile to prepare the solution of oxidized glutathione with a concentration of 3 mM (0.01 %), and the RNA purity after the experiment was measured. The results are shown in Table 7.

[Comparison Example 5]

One hundred (100) μL of the RNA solution that was obtained by a preparative purification by a reversed-phase column chromatography in the Reference Example 3 was placed in a volume 300 mL of polypropylene vial (manufactured by Thermo Fisher Scientific), and the vial containing the solution was placed in an incubator (manufactured by Kenis Limited) in which the temperature was adjusted to 60°C, and the vial was left to stand for 14 hours. After left to stand, the polypropylene vial taken out from the incubator was cooled to room temperature, and the purity was calculated by

the method described in the above-mentioned measurement method 1. The results are shown in Table 7.

[Comparison Example 6]

The experiment was carried out under a similar condition to those of the Exmaple 18 wherein each of a solution of isobutylene sulfide in acetonitrile or a solution of 4-tert-butyl diphenyl sulfide in acetonitrile was used as an additive solution in a prescribed concentration respectively instead of the solution of lipoic acid in acetonitrile. The results are shown in Table 7.

[Table 7]

| | Additives | Concentration of Additives (mM) | Purity by HPLC measurement 1) | Retention ratio of Nucleic acid 2) |
|---|---|---|---|---|
| Ex. 18 | α-lipoic acid | 3.0 | 91.9 % | 97 % |
| Ex. 19 | L-methionine | 3.0 | 91.4 % | 96 % |
| Ex.20 | DL-methionine | 3.0 | 92.3 % | 97 % |
| Ex. 21 | Oxidized glutathione | 0.15 | 91.0 % | 96 % |
| Comparative Ex.5 | no additive | - | 88.8 % | 93 % |
| Comparative Ex.6 | Isobutylene sulfide | 3.0 | 31.7 % | 33 % |
| | 4-tert-butyl diphenyl sulfide | 3.0 | 85.0 % | 89 % |

1) which represents a purity of nucleic acid that was obtained by leaving the nucleic acid (Purity 95.1 %) prepared in the Reference Exmaple 3 to stand at 60°C for 8 hours.
2)

Retention ratio of nucleic acid (%) = Nucleic acid purity after left to stand / Nucleic acid purity before left to stand

[Industrial Applicability]

According to the present invention, a stable composition comprising a nucleic acid oligomer having a phosphorothioate bond can be obtained, and accordingly the composition can be effectively prepared.

[Sequence Listing Free Text]

Sequence Nos. 1 and 2 in a sequence listing represent a base sequence of oligonucleotide that is prepared according to the process of the present invention.

## Claims

**1.** A composition comprising a nucleic acid oligomer having a phosphorothioate bond represented by formula (1):

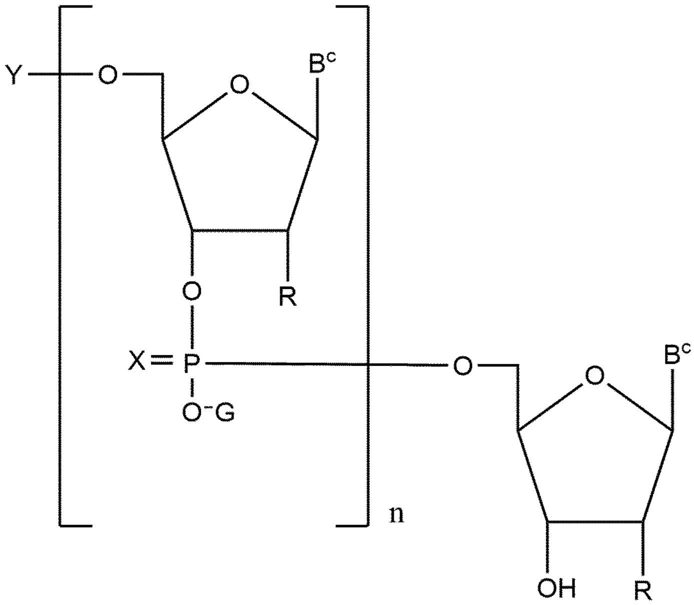

(1)

wherein

$B^c$ is the same or different from each other and each represents independently a nucleic acid base,
R is the same or different from each other and each represents independently a hydrogen atom, a fluorine atom, or OQ group,
Q is the same or different from each other and each represents a hydrogen atom, a methyl group, a 2-methoxyethyl group, a methylene group that is attached to a carbon atom at a 4'position of ribose, an ethylene group that is attached to a carbon atom at a 4'position of ribose, or an ethylidene group that is attached to a carbon atom at a 4'position of ribose,
X is the same or different from each other and each represents an oxygen atom or a sulfur atom,
Y represents a hydrogen atom or a protecting group of hydroxy group,
G represents an ammonium ion, an alkylammonium ion, an alkali metal ion, a hydrogen ion or a hydroxyalkylammonium ion,
n is an integer that satisfies an equation (2):

$$15 \leqq n \qquad (2),$$

an alkylammonium salt, a water-soluble organic solvent, water, and an additive,
the additive is an additive including at least one compound selected from the group consisting of compounds represented by the following formula (3) or (4),
a compound represented by formula (3):

$R^a(R^c)CH$-L-$CH(R^d)R^b$ (3)

wherein

L represents -S- or -SS-,
$R^a$ and $R^b$ are the same or different from each other and each represents independently a hydrogen atom, or a C1-6 alkyl group which may be optionally substituted with at least one group selected from a group consisting of the following $Z^1$ and $Z^2$,

$Z^1$: $-CH(NHR^1)COR^2$,

$Z^2$: $-COR^2$

in $Z^1$ and $Z^2$,

$Z^1$ represents a hydrogen atom, a protecting group for amino group, or C(O)-$R^{11}$ group,
$R^{11}$ represents a C1-6 alkyl group which may be optionally substituted with at least one group selected from a group consisting of amino group and carboxy group, or a phenyl group which may be optionally substituted with at least one group selected from a group consisting of amino group and carboxy group,
$R^2$ represents a C1-6 alkylimino group which may be optionally substituted with carboxy group, wherein the carboxy group may be optionally protected, or $-OR^{20}$ group ($R^{20}$ represents a hydrogen atom or a protecting group for carboxy group),
$R^e$ and $R^d$ are the same or different from each other and each represents independently a hydrogen atom, or a C1-6 alkyl group,
a compound represented by formula (4):

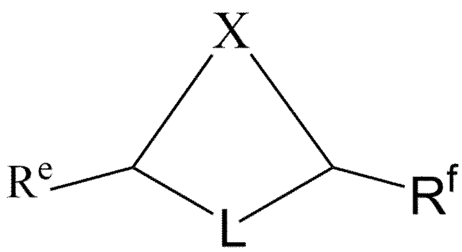

(4)

wherein
L has the same meanings as defined above,
$R^e$ and $R^f$ are the same or different from each other and each represents independently a hydrogen atom, a C1-6 alkoxycarbonyl group, a carboxy group, or a C1-6 alkyl group which may be optionally substituted with C1-6 alkoxycarbonyl group or carboxy group,
X, L and carbon atoms to which they are attached form a 5- membered or 6- membered ring structure, and
X represents any group selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C$=N, $CH_2NH$, $(CH_3)CHNH$, $(CH_3)_2CNH$, (COOH) CHNH, $CH_2OCH_2$, $CH_2NHCH_2$ and $CH_2COCH_2$.

**2.** The composition according to claim 1 wherein

$R^1$ represents a hydrogen atom, a protecting group for amino group, or C(O)-$R^{11}$ group,
$R^{11}$ represents a C1-6 alkyl group that may be optionally substituted with at least one group selected from the group consisting of amino group and carboxy group, and
X represents any groups selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C$=N, $CH_2NH$, $(CH_3)CHNH$, (COOH) CHNH, $CH_2OCH_2$, and $CH_2COCH_2$.

**3.** The composition according to claim 1 or 2 wherein

$R^1$ represents a hydrogen atom, a benzoyl group, a 4-methoxybenzoyl group, a formyl group, an acetyl group,

a propionyl group, a butyryl group, an isobutyryl group, a phenylacetyl group, a phenoxyacetyl group, a 4-tert-butyl phenoxyacetyl group, a 4-isopropyl phenoxyacetyl group, a benzyloxy carbonyl group, a 9-fluorenylmethyloxy carbonyl group, or C(O)-$R^{11}$ group,
$R^{11}$ represents a C1-6 alkyl group that may be optionally substituted with at least one group selected from the group consisting of amino group and carboxy group,
$R^2$ represents a C1-6 alkylimino group that may be optionally substituted with carboxy group wherein carboxy group may be protected by methyl group, benzyl group, allyl group or tert-butyl group, or -$OR^{20}$ group ($R^{20}$ represents a hydrogen atom, a methyl group, a benzyl group, an allyl group or a tert-butyl group), and
X represents any groups selected from $CH_2$, $CH_2CH_2$, $(CH_3)CHCH_2$, $(CH_3CH_2)CHCH_2$, $CH_2CH_2CH_2$, $(CH_3)CHCH_2CH_2$, $CH_2(CH_3)CHCH_2$, CH=N, $(CH_3)C=N$, $CH_2NH$, (COOH)CHNH, $CH_2OCH_2$, and $CH_2COCH_2$.

**4.** The composition according to any one of claims 1 to 3 wherein

$R^e$ and $R^f$ are the same or different from each other and each independently represents a hydrogen atom, a carboxy group, or a C1-6 alkyl group, and
X represents any groups selected from $CH_2$, $(CH_3)C=N$, $CH_2NH$, $CH_2OCH_2$, and $CH_2COCH_2$.

**5.** The composition according to any one of claims 1 to 4 wherein

$R^1$ represents a hydrogen atom, a benzoyl group, a formyl group, an acetyl group, a benzyloxy carbonyl group and a 9-fluorenylmethyloxy carbonyl group or C(O)-$R^{11}$ group,
$R^{11}$ represents a C1-6 alkyl group that may be optionally substituted with at least one group selected from the group consisting of amino group and carboxy group, and
$R^2$ represents a C1-6 alkylimino group that may be optionally substituted with carboxy group wherein the carboxy group may be optionally protected by methyl group, or -$OR^{20}$ group ($R^{20}$ represents a hydrogen atom or a methyl group) .

**6.** The composition according to any one of claims 1 to 5 wherein

the additives are at least one compound selected from the group consisting of

α-lipoic acid,
methionine,
N-formyl methionine,
N-acetyl methionine,
N-benzoyl methionine,
N-carbobenzoxy methionine,
N-Fmoc-methionine,
methionine methyl ester hydrochloride salt,
dibutyl sulfide,
dihexyl sulfide,
thiazolidine-2-carboxylic acid,
2-isobutyl-4,5-dimethyl-3-thiazoline (isomer mixture),
4-oxothiane,
1,4-thioxane, and
oxidized glutathione.

**7.** The composition according to any one of claims 1 to 6 wherein the additives are at least one compound selected from the group consisting of lipoic acid, oxidized glutathione and methionine.

**8.** The compound according to any one of claims 1 to 7 wherein the alkylammonium salt is at least one of alkylammonium salt selected from the group consisting of monoalkylammonium salts and dialkylammonium salts.

**9.** The composition according to any one of claims 1 to 8 wherein the water-soluble organic solvent is a water-soluble organic solvent selected from the group consisting of alcoholic water-soluble organic solvents and nitrile water-soluble organic solvents.

**10.** The composition according to any one of claims 1 to 9 wherein in the above formula (1), R each independently

represents a hydroxy group or a methoxy group.

**11.** The composition according to any one of claims 1 to 9 wherein in the above formula (1), R represents a hydroxy group.

**12.** A process for preparing a nucleic acid oligomer which comprises a step of mixing the composition according to any one of claims 1 to 11 with a C1-C4 organic solvent containing at least one oxygen atom to isolate a precipitated nucleic acid oligomer.

**13.** A process for preparing the composition according to any one of claims 1 to 12 which comprises a step of mixing column eluate containing a nucleic acid oligomer represented by formula (1) wherein the nucleic acid oligomer is obtained by subjecting a crude product of a nucleic acid oligomer of formula (1) that is synthesized by a solid-phase synthesis method to a reversed-phase chromatography, an alkylammonium salt, a water-soluble organic solvent and water,
with an additive.

[Fig. 1]

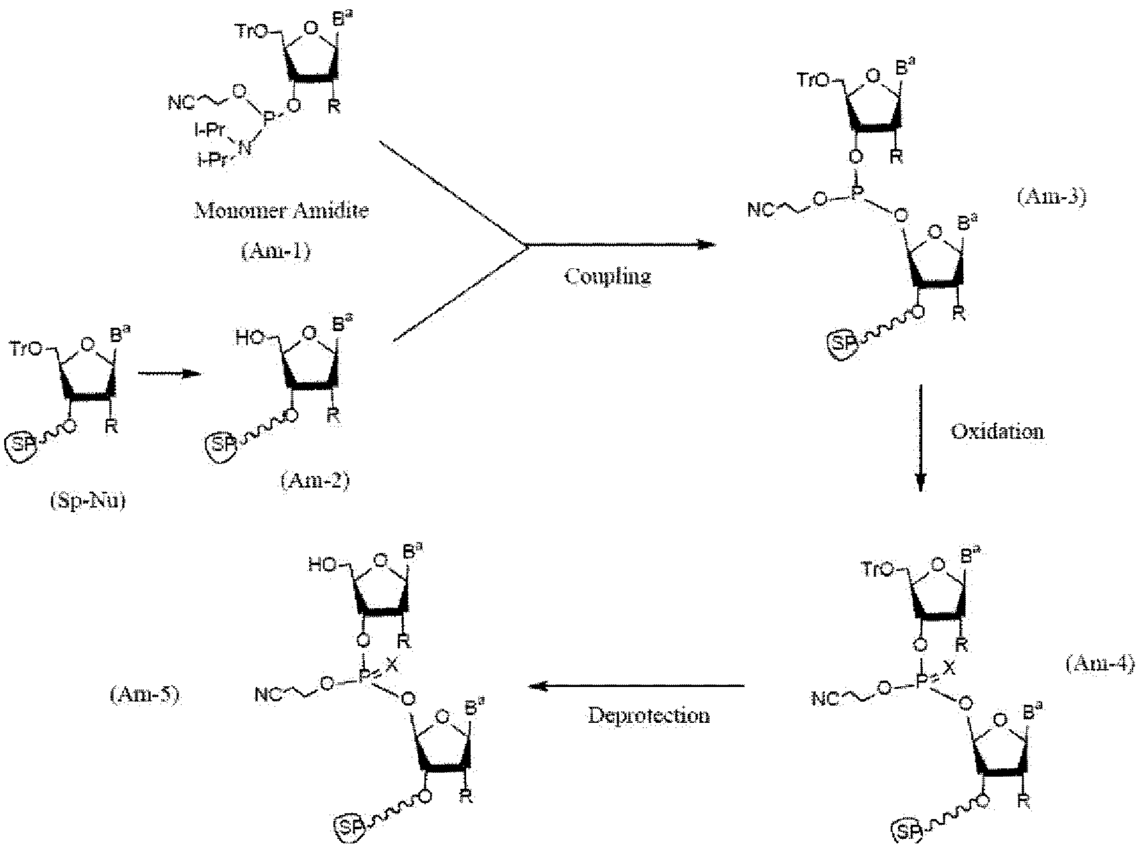
Monomer Amidite
(Am-1)
Coupling
(Am-3)
(Sp-Nu)
(Am-2)
Oxidation
(Am-4)
Deprotection
(Am-5)

**INTERNATIONAL SEARCH REPORT**

International application No.
PCT/JP2021/014017

A. CLASSIFICATION OF SUBJECT MATTER
Int.Cl. C07H21/02(2006.01)i, C07H21/04(2006.01)i, C12N15/09(2006.01)i
FI: C07H21/02, C07H21/04A, C12N15/09

According to International Patent Classification (IPC) or to both national classification and IPC

B. FIELDS SEARCHED

Minimum documentation searched (classification system followed by classification symbols)
Int.Cl. C07H21/02, C07H21/04, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 03/005822 A1 (ISIS PHARMACEUTICALS, INC.) 23 January 2003 (2003-01-23), tables 3, 4, page 25, lines 11-23, page 36, line 28 to page 37, line 12 | 1-11<br>12, 13 |
| A | JP 7-170981 A (RES DEV CORP OF JAPAN) 11 July 1995 (1995-07-11), claims | 1-13 |
| A | JP 2003-531827 A (ISIS PHARMACEUTICALS INC.) 28 October 2003 (2003-10-28), claims | 1-13 |
| A | WO 2018/141908 A1 (GENE SIGNAL INTERNATIONAL SA) 09 August 2018 (2018-08-09), claims | 1-13 |
| P, A | WO 2020/196890 A1 (AJINOMOTO CO., INC.) 01 October 2020 (2020-10-01), claims | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May 2021 | 01 June 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/014017

| | | |
|---|---|---|
| WO 03/005822 A1 | 23 January 2003 | US 2003/0096770 A1<br>tables 3, 4, paragraphs [0069], [0103]<br>US 2005/0208528 A1<br>CA 2451776 A1 |
| JP 7-170981 A | 11 July 1995 | (Family: none) |
| JP 2003-531827 A | 28 October 2003 | US 6586586 B1<br>claims<br>US 2003/0153742 A1<br>WO 2001/055160 A1<br>CA 2398749 A1<br>KR 10-2003-0032915 A |
| WO 2018/141908 A1 | 09 August 2018 | US 2018/0221401 A1<br>claims<br>US 2019/0350963 A1<br>JP 2020-515525 A<br>CN 110520134 A<br>CA 3051724 A1 |
| WO 2020/196890 A1 | 01 October 2020 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020072234 A
- WO 2017068377 A1
- US 3687808 A
- JP 4476386 B
- JP 5317836 B
- JP 5157168 B
- JP 5554881 B
- WO 2013027843 A1
- WO 2006022323 A1
- WO 2019208571 A1


### Non-patent literature cited in the description


- Concise Encyclopedia Of Polymer Science And Engineering. John Wiley & Sons, 1990, 858-859
- **ENGLISCH et al.** *Angewandte Chemie, International Edition,* 1991, vol. 30, 613
- Fundamentals of modern peptide synthesis. **MURIEL AMBLARD ; JEAN-ALAIN FEHRENTZ ; JEAN MARTINEZ.** Methods in Molecular Biology (Trademark). book series, 2005, vol. 298, 3-24
- Handbook of Analysis of Oligonucleotides and Related Products. CRC Press
- Reviews by Chakhmakhcheva: Protective Groups in the Chemical Synthesis of Oligoribonucleotides. *Russian Journal of Bioorganic Chemistry,* 2013, vol. 39 (1), 1-21